Europäisches Patentamt

European Patent Office

Office européen des brevets

(11) Publication number: **0 352 955**
**A2**

## EUROPEAN PATENT APPLICATION

(21) Application number: 89307296.7

(22) Date of filing: 19.07.89

(51) Int. Cl.⁴: **A61B 17/22 , A61M 25/00**

(30) Priority: 22.07.88 US 223467

(43) Date of publication of application:
**31.01.90 Bulletin 90/05**

(84) Designated Contracting States:
**DE FR GB**

(71) Applicant: **BAXTER INTERNATIONAL INC.**
**One Baxter Parkway**
**Deerfield, IL 60015(US)**

(72) Inventor: **Son-Chou, Jim**
**19502 Sierra Mia Road**
**Irvine, CA 92715(US)**
Inventor: **Chen, Peter**
**12 Alegria**
**Irvine, CA 92714(US)**
Inventor: **Martinez, Richard**
**397 Poplar Street**
**Laguna Beach, CA 92651(US)**
Inventor: **Ho, Daniel N.**
**445 Yellowstone Circle**
**Corona, CA 91719(US)**
Inventor: **de la Rama, Alan Diaz**
**11315 Selson Street**
**Cerritos, CA 90701(US)**
Inventor: **Nguyen, Tho H.**
**9441 Keolki Circle**
**Huntingdon Beach, CA 92646(US)**

(74) Representative: MacGregor, Gordon et al
**ERIC POTTER & CLARKSON 14 Oxford Street**
**Nottingham, NG1 5BP(GB)**

(54) Metal hot tip catheter with fused tip and temperature feedback loop.

(57) Disclosed is a hot tip catheter having minimal tip dissociation, which reaches and consistently maintains very high temperatures. The catheter body is formed of spirally wound metal wire (2) which is relatively flat, each spiral substantially in contact with the next, to improve column strength, and is sealed with teflon (21) and a distal collar (22). The hot tip (3) is also formed of metal and is welded to the catheter body to eliminate dissociation of the tip. Heating is a accomplished by a catalytic reaction of $H_2$ and $O_2$ at the tip monitored via a thermocoupler, and the catheter body includes a gas inlet lumen (connected to a gas generator) and a vacuum outlet lumen (connected to a vacuum pump) to input the gas and remove the heat by-products, respectively. A temperature control loop is preferably included which regulates the inflow of gas to the catheter, and the temperature of the tip.

FIG. 1

## METAL HOT TIP CATHETER WITH FUSED TIP AND TEMPERATURE FEEDBACK LOOP

### BACKGROUND OF THE INVENTION

#### Field of the Invention

This invention relates to catheters, in particular hot tip catheters for use for angioplasty and related functions.

#### Description of the Prior Art

A catheter initally was considered a tubular surgical instrument for withdrawing fluids from or introducing fluids into a body cavity. Recent catheters, however, perform many more functions than the handling of fluids, including angioplasty, dilatation, and the like. A catheter can be inserted directly, but is frequently inserted by slipping it over a smaller guidewire which has been pre-inserted to location in a blood vessel or the like. The guidewire is usually made of metal, and may be spirally wound part of its length.

Commonly, the catheter itself is composed of teflon tubing or another biologically inert polymer material. Once in a while, the teflon tubing is reinforced by metal, for example loosely wound stainless steel or copper wire which helps the tubing resist lateral stress, as disclosed in U.S. patents No. 4,619,643, and 4,577,543. Such wire is usually inserted in the mold with the tubing or wrapped around the tubing during manufacture of the catheter. Catheters themselves however have apparently never been made of metal because it has been thought that they would be insufficiently torquable and have insufficient column strength to be effectively steered into difficult areas along winding and tortuous vessel pathways.

Hot tip catheters, specifically, are known and used for angioplasty and frequently need to be steered to difficult occlusion sites in the vascular system. Current hot tip catheters generally include a catheter body made of teflon crimped into a metal tip which is heated in order to soften or even melt plaque in a blood vessel to reform the plaque against the vessel walls and eliminate occlusions. In current catheters, the metal tip is heated electrically, or by a laser beam transmitted via a fiberoptic cable.

A common problem with such catheters is that teflon (or any other common polymer used for a catheter body) softens and even melts upon the application of heat. Needless to say, the temperature of the catheter tip is thus limited, usually to about 250 to 300°C, and can be used for angioplasty, but not for any functions (for example true cauterizing, or discectomies) which may require higher temperatures, or the actual vaporization of tissue.

A further problem, commonly reported in the literature, is that even at such limited temperatures, the teflon of the catheter body softens enough that it can and sometimes does dissociate from the tip. Normally, to prevent the tip from being completely lost in the vessel if it dissociates, a wire is welded to the tip and extends back through the vessel next to, but exterior to, the catheter. The wire can sometimes also be used to steer the catheter. However, use of the wire to remove a dissociated tip is cumbersome and poses a risk of damage to the vessel walls. In addition, it is possible that the single spot weld attaching the tip to the wire may itself break while attempting to remove the tip. Needless to say, it would be desireable to minimize or eliminate the tip dissociation risk entirely.

### SUMMARY OF THE INVENTION

The present invention is an apparatus such as a catheter and a flow control loop, both with many improved characteristics. In one aspect, the invention includes a catheter having a tip bonded, fused, or welded to the catheter body to minimize dissociation of the tip from the body. In the preferred embodiment, a hot tip catheter, both the tip and the catheter are formed of metal and are welded together to minimize tip dissociation, a common problem with prior hot tip catheters. This catheter can be used for angioplasty, cauterizing, discectomies and other functions and the tip can reach very high temperatures (i.e. 800°C.) if necessary. The catheter also preferably includes a gas inlet lumen, a catalyst in the tip which is self-igniting in the presence of appropriate gas to produce an exothermic reaction to heat the tip, a vacuum outlet lumen for removing by-products of the catalyzed reaction, and a thermocouple for reading the tip temperature.

In another aspect, the catheter includes a metal body portion, preferably formed of spirally-wound stainless steel wire, most preferably rectangular and sufficiently tightly wound so that adjacent spirals are substantially in contact with each other along the length of the catheter. Because the body is metal, it can be welded to a metal tip such as that used in a hot tip catheter. Further, the spiral windings allow flexibility in the catheter, yet provide sufficient column strength to, in many instances, allow the catheter to be inserted without a guidewire.

In another aspect, the invention includes a flow-control loop for controlling flow, as of a fluid or gas, in response to an external condition. The loop includes means for reading the external condition, a pathway between the flow source and the flow output, and means for variably controlling the flow through the pathway in response to readout of the external condition from the reading means. The external condition, naturally, is usually the temperature of a catheter, particular the hot tip of a catheter such as the one described herein.

Preferably, the loop includes a means for reading the temperature of the tip such as a thermocouple, a microcontroller responsive to the temperature readout, a pathway between a gas source and the catheter, a needle valve to variably open or close the pathway via a stepper motor run by the microcontroller, and a three-way solenoid valve to close the pathway when the temperature becomes too high. Most preferably, a dump valve is included to purge the pathway of gas when the system is completely overheated, as is a second pathway parallel to the main pathway and controlled by a three-way solenoid pulsed open and closed at a given rate by the microcontroller when necessary to increase temperature. With this temperature control loop, the tip of a hot tip catheter such as the catheter described herein remains at a reasonably stable, pre-set temperature without constant operator intervention, by regulating the flow of gas to the tip and the exothermic reaction there. It also aborts heating and the entire procedure if and when the tip overheats.

## BRIEF DESCRIPTION OF THE DRAWINGS

Fig. 1 is an elongate sectional view of the distal portion of the catheter of the present invention, taken at lines 1-1 of Fig. 4.

Fig. 2 is a cross section of the distal portion of the preferred catheter, taken at lines 2-2 of Fig. 1.

Fig 3 is an elongate sectional view of the tip of the preferred catheter.

Fig. 4 is a plan view of the preferred catheter of the present invention.

Fig. 5 is a flow chart of the software controlling the preferred temperature control loop of the present invention.

Fig. 6 is a flow chart of the temperature control loop and catheter in use in the present invention.

## DESCRIPTION OF THE SPECIFIC EMBODIMENTS

In this specification, and the claims which follow, the words "bond" and conjugated terms (i.e. "bonded," or "bonding") mean attaching two surfaces to each other via the same or a different material, such as by using adhesives, or by welding.

"Column strength" refers to the ability of an elongate unit to retain its integrity as a column under pressure.

"Torquability" refers to the ability of a column to translate circumferential motion about the central longitudinal axis which is applied at one end of the column, to the far end of the column.

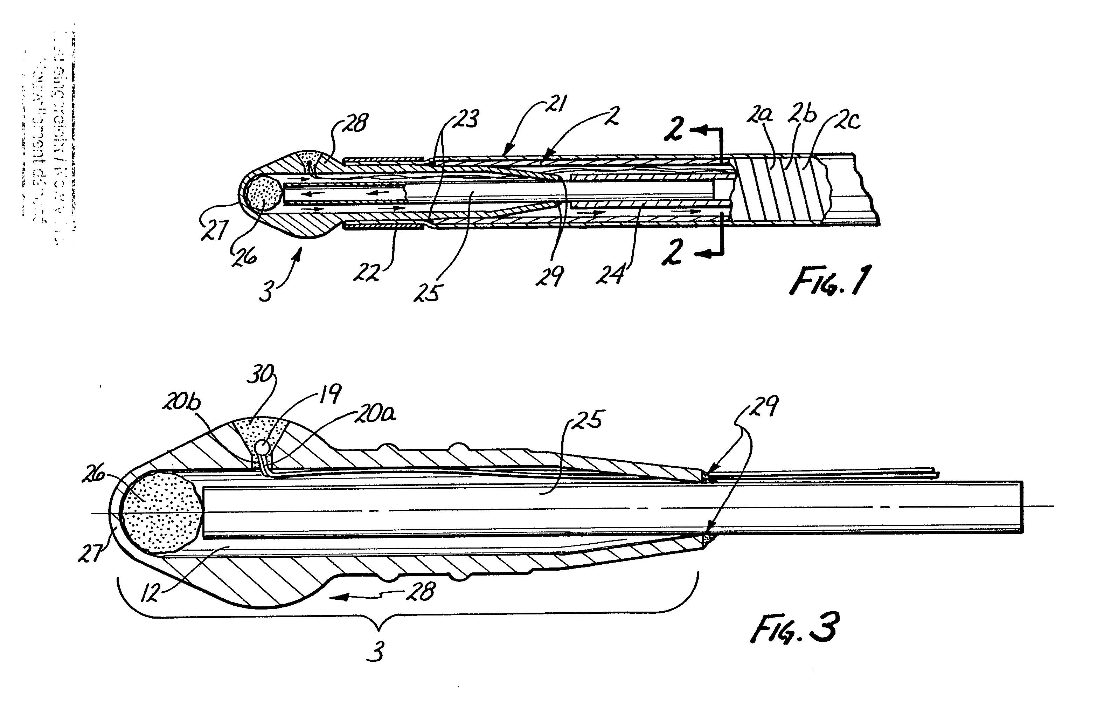

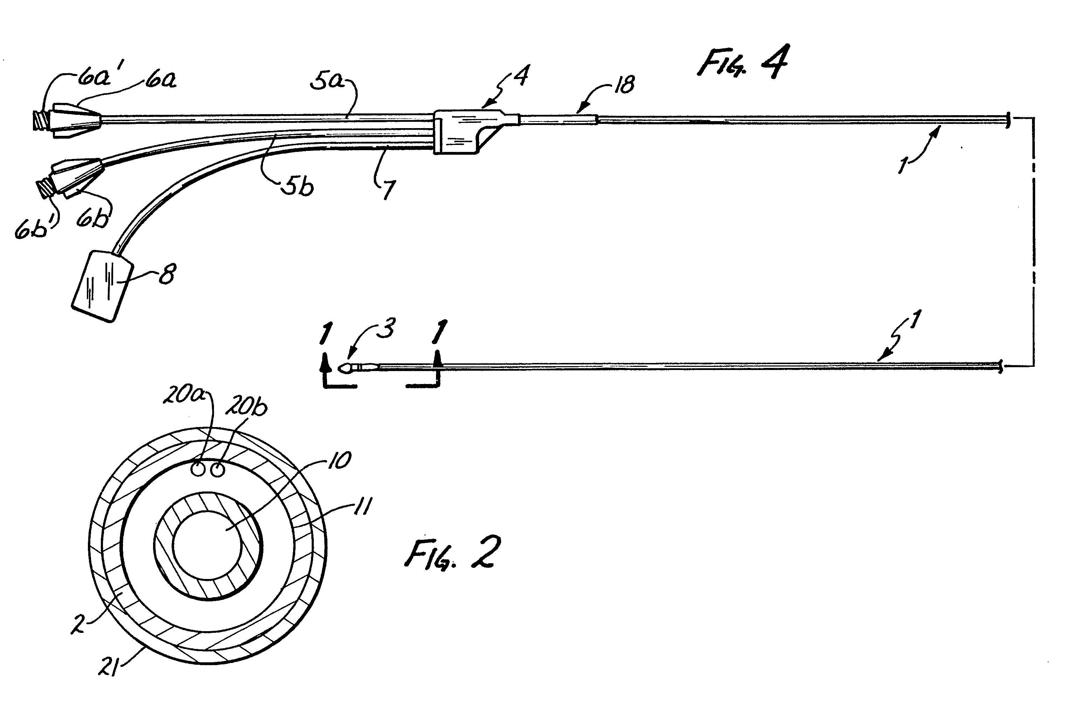

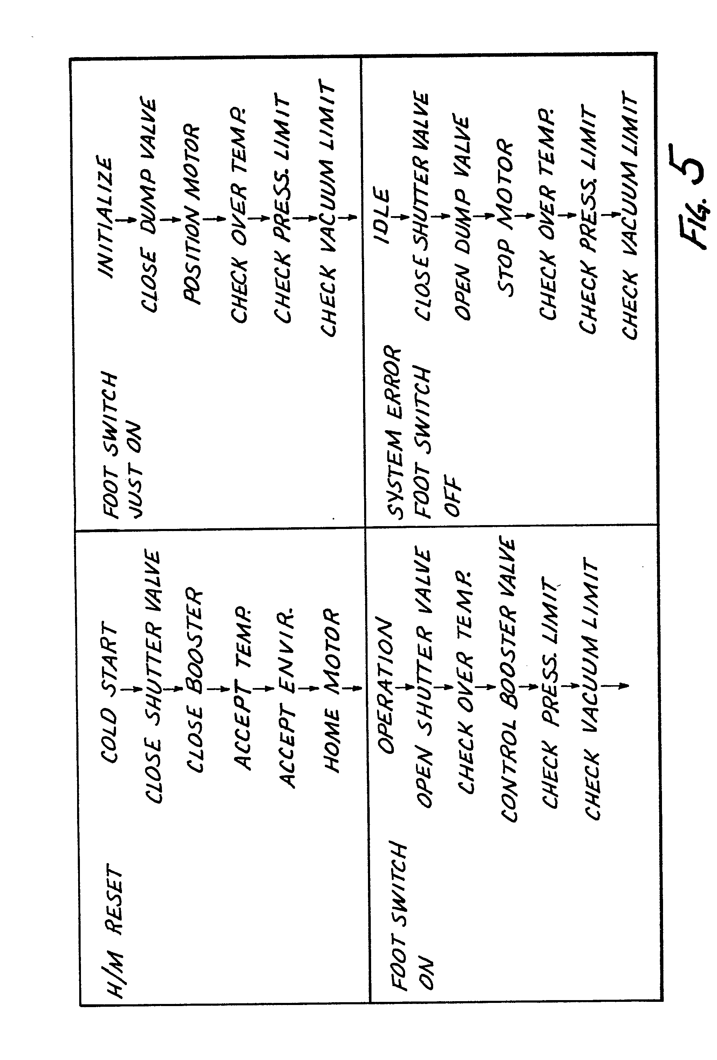

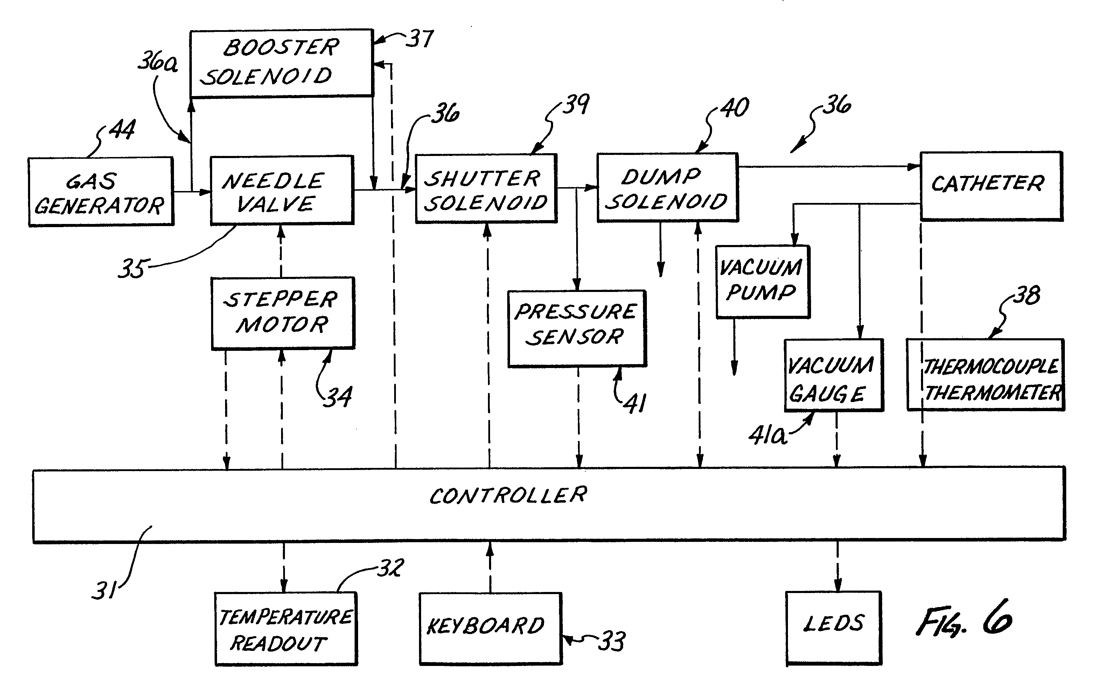

Referring first to Fig. 4, the hot tip catheter of the present invention includes a tubular catheter body 1 preferably about a meter long and 1.6 mm (5 fr.) in exterior diameter, terminating at the distal end in a tip 3. As shown in Fig. 2, the catheter body contains two coaxially-disposed lumens 10 and 11. the former within the latter.

Catheter body 1 terminates proximally in a polyvinyl chloride ("PVC") adapter 4 bonded with a vinyl adhesive to PVC tubing 18 heat shrunk around the catheter body. Inner lumen 10 extends through adapter 4 and out through extension tube 5a where a PVC gas input hub 6a is bonded to it; an epoxy adhesive seal is provided at the exit from adapter 4. Gas input hub 6a contains a luer lock 6a' for attachment to a gas generator 44 (see Fig. 6) such as a Henes Products Corporation electrolytic gas generator producing gas (preferably $H_2$ and $O_2$) from distilled water or other solutions at pressures of up to about 25 psi, preferably up to about 15 psi.

Thermocouple leads 20a and 20b (shown in Figs. 1, 2 and 3) extend through adapter 4 into extension tube 7 (also sealed to the adapter with epoxy adhesive) adhesively attached to thermocouple connector 8. Extension tube 5a exits directly from adapter 4 and terminates proximally in a vacuum hub 6b with a luer lock 6b' attached to a vacuum pump 42 operating in the range of about -20 to -29.9 inches Hg, preferably about -25 inches Hg to -29.8 inches Hg, for aspirating by-products of heating from the catheter.

As shown in Fig. 1, catheter body 1 is preferably formed of a material with a melting point above about 200°C, such as metals, ceramics, polyamides, or carbon. It is preferably metal, most preferably stainless steel. In the preferred embodiment, it consists of a spirally-wound, relatively flat, stainless steel wire 2 sufficiently tightly wound so that adjacent spirals (e.g. 2a, 2b, and 2c) are substantially in contact with each other. The wire is preferably about .016" wide and .004" thick (available from Acme String Inc as Type 302 or 304 stainless rectangular wire), but can be round if desired. It is wrapped about a mandril to an internal diameter of about 0.055 inches in the preferred embodiment by procedures known in the art. The spirally wound body is thought to improve flexibility of the catheter, and the tightly wound configuration with flat wire to particularly improve its column strength.

The catheter body is jacketed with teflon heat-shrunk tubing 21 which forms a gas-tight seal for the catheter, and helps hold the spiral windings of the catheter body (e.g. 2a, 2b, and 2c) in place. The jacket is sealed at the distal end preferably by a collar 22 (99% silver) crimped around it, also shown in Fig. 1.

In the preferred embodiment, the stainless steel body 2 actually terminates proximal to collar 22; tip 3 is inserted into body 1 and collar 22 in fact crimps jacket 21 about the tip. Collar 22 has about the same exterior diameter as catheter body 1 sealed with jacket 21.

Tip 3 is preferably slightly bulbous (see area 28 on Figs. 1 & 3) immediately adjacent the collar 22, but terminates in a narrower, rounded end portion 27 to form a substantially conical tip having an internal lumen 12 slightly smaller in diameter than the internal diameter of the catheter body. The tip 3 in most embodiments should be made of material having a melting point above about 200°C, such as metals, ceramics, polyamides, or carbon. Tip 3, preferably 1.5 to 2.0 mm. in diameter at its widest portion, and preferably of metal, is machined of stainless steel by procedures known in the art.

Lumen 10 is formed by teflon tubing 24 about 0.018" in internal diameter which extends into the distal portion of the catheter but not as far as the collar 22 or tip 3. At its distal end, tubing 24 receives metal, preferably stainless steel, tubing 25. Tubing 25 is sufficiently long (preferably about 10 mm.) to be stably received (usually for about 3 mm.) within tubing 24 (usually for about 3 mm.) and terminates within tip 3 immediately adjacent catalyst 26 placed distally within lumen 12 of tip 3.

Catalyst 26 is preferably formed of an alumina ceramic impregnated with platinum or palladium nitrate such that it is self-igniting. A detailed description of a typical catalyst 26 is found in U.S. Patent Application Serial No. 07/093,027 entitled Catheter and Method for Providing Thermal Energy To Same and filed September 4, 1987, which is hereby incorporated herein by reference as though fully set forth.

Tip 3 is attached to catheter body 20 via cylindrical weld 23 at the distal end of the catheter body, and to tubing 25 in two spot welds 29 just adjacent to tubing 24. A thermocouple 19, preferably type K (i.e. reading from 0 to about 1200°C.) is welded in the bulbous area 28 of tip 3 and is attached to insulated leads 20a and 20b (chromel and alumel, respectively.) Leads 20a and 20b extend the length of the catheter to the thermocouple connector 8 and ultimately to thermocouple thermometer 38 where the temperature of the tip is determined.

Thermocouple connector 8 is attached proximally to a temperature control system which maintains the hot tip 3 substantially at a pre-set temperature. As shown in Fig 6, the system includes the feedback from the thermocouple fed to a thermocouple thermometer 38 such as an Omega Model 660. Data converted by the thermometer is fed serially to microcontroller 31 (preferably an Intel 8752) which has a temperature readout 32, an overtemperature light, other desirable readouts and a keyboard or key input 33 for, for example, input to set the desired tip temperature.

Microcontroller 31 delivers and receives electronic output and input signals to control a stepper motor 34 preferably having fairly small steps which in turn controls a one-way, fine metering needle valve 35 which is in the path of and controls the flow of gas from the gas generator 44 via main pathway 36 (of nylon tubing) to the catheter.

Microcontroller 31 also provides on/off control to a three-way booster solenoid valve 37 governing a separate gas pathway 36a parallel to main pathway 36, on/off control to a three-way "shutter" solenoid 39 in the main pathway 36, and on/off control to a three-way "dump" solenoid 40 also in the main pathway 36 whose function is to dump and purge gas from the system in case of overtemperature. In some embodiments, only the shutter solenoid valve is provided in the loop; it is controlled in an on/off fashion by the microcontroller, closing when the temperature is too high (to prevent gas from passing to the catheter) and opening when the temperature is in the correct range; in others, only the needle valve is provided. However, such systems do not seem to be as effective as the preferred embodiment in maintaining temperature control.

Microcomputer 31 receives input from a pressure sensor or transducer 41 in the main pathway and from a vacuum transducer 41a between the vacuum pump and the catheter. All solenoid, valves, and motors in the temperature control system can be easily be purchased. Software is provided for appropriate control of the temperature feedback system, is shown in Fig. 5, and will be described in greater detail during the description of operation below.

To calibrate the system before the first use, or to recalibrate later, calibration mode is selected. The desired temperature or temperatures are input from the keyboard, and the position of the needle valve repeatedly adjusted by the stepper motor until the desired temperature of the tip stabilizes for about 30 seconds. This baseline information together with the environmental conditions such as the environment--i.e. blood or air is saved in an EEPROM for later use when the system is in formal operation. To use the system, the catheter is inserted into the vascular system adjacent the plaque deposit to be restructured, reformed, or removed. A guidewire has not been found to be necessary for catheter insertion because the catheter itself has sufficient torquability and column strength to be inserted through the vascular system by itself. This is believed to be the result of the spirally-wound catheter body, particularly when flat wire is used which is sufficiently tightly wound to render adjacent spirals substantially in contact with each other. Needless to say, however, in other embodiments, a guidewire, (and thus an open-ended tip) may be necessary; in such case, the guidewire is first inserted, the catheter then inserted over it and the guidewire removed.

It should be understood here that the present catheter can be operated as is, i.e. manually with only the gas generator and vacuum pump and without any temperature feed-back loop. However, it has been found difficult to maintain consistent temperature manually, and the temperature feedback loop is preferred.

If the feedback loop is used, the gas generator and vacuum pump are attached to to the controller 41, the catheter then connected, and the system then turned on. This powers up the vacuum pump and lights the indicators showing the temperature and the pressure in the system. The desired temperature is selected and input to the microcontroller, the microcontroller reads the nature of the environment and sets the needle valve to the encloded position for the temperature, thus setting the desired gas flow rate for the temperature (usually less than about 20 cc. per minute).

When the operator is ready to begin, as shown in Fig. 5, the foot pedal is depressed which closes the dump valve, positions the stepper motor, and checks to see that the temperature, pressure, and vacuum are not overlimit. The shutter solenoid valve is then opened, allowing gas flow. Gas ($H_2$ and $O_2$)) is then fed to the catheter tip through lumen 10 where it exits immediately adjacent the catalyst 26 for a highly exothermic reaction producing water vapor. This reaction heats the end portion (27) of tip 3 which is then directed by the operator around the plaque in order to reform or restructure it against the vessel walls to eliminate the occlusion. The larger, bulbous side (28) of the tip, due to its larger mass, does not heat up as much as the end portion to minimize inadvertent damage to the vessel wall. Furthermore, because the tip is welded to the catheter body, the chances of tip dissociation are minimal, and the procedure can be freely used without concern by the physician. Vacuum pump 42 aspirates the water vapor (and any other by-products) from the system through lumen 11. Preferred vacuum pressures are about -25 to -29.8 inches Hg.

During the angioplasty process (which may take an hour or so), the thermocouple 19 repeatedly reads the tip temperature, sending messages via leads 20a, 20b to the microcontroller 31 to control the tip temperature. To maintain the safety of the system, if the temperature is more than a pre-set temperature (450°C in the preferred embodiment) the dump valve is opened to purge the system, the shutter valve closed to prevent further input, and the overtemperature light lit, so that the procedure can be aborted, and the catheter removed once it is cool enough to do so.

To maintain temperature in the desired range, however, when the temperature is more than 10°C. over the desired temperature, but not at the abort temperature (450°), the shutter valve is closed to close off all the gas flow to the catheter until the temperature drops to the desired temperature. If the temperature is more than 10°C below the desired temperature, the booster valve is opened in pulses to allow more gas into the system parallel with the gas passing through the needle valve. The duration of the signal to the booster valve is proportional to the difference between the desired and actual temperature.

Once the procedure is complete, the system is turned off, and the cooled catheter removed.

To manufacture the catheter of the present invention, the tip is first machined and the catalyst inserted. The catheter body is formed, and the inner lumen 10 and thermocouple 19 and leads 20a and 20b passed through it. Thermocouple 19 is hand-welded into place on the tip. Tubing 25 is inserted (finger-tight) into inner lumen 10 and at the other end into tip 3, which is then hand-welded in two spots 25 onto place; the catheter body 1 is then also hand-welded in a ring 23 to the tip 3. Teflon tubing of the appropriate size is then fit around the catheter body adjacent the bulbous area of the tip 3 and heat shrunk into place. Collar 22 is slid from the proximal end of the catheter into place and crimped with a crimping tool.

The teflon jacket is then removed from the last inch or so of the proximal end of the catheter body 2 and about 1 and 1/2 inches of PVC tubing of the appropriate size slid on from the proximal end. Epoxy adhesives are applied to the surface of the teflon and stainless steel at their juncture and the PVC tubing heat shrunk into place over the adhesive. PVC adapter 4 is slid into place over the inner lumen 10 of the catheter body and is bonded by vinyl adhesives. The inner lumen 10 is threaded through the proximal end of the adapter and through PVC extension tube 5a which is sealed at the exit from the adapter with epoxy adhesive. Leads 20a and 20b are also threaded through extension tube 7 which is also sealed at the exit with epoxy adhesive. Finally, extension tube 5b is inserted into the adapter directly as a vacuum tube and also sealed with epoxy adhesive. The gas input hub 6a and the vacuum outlet hub 6b are attached to the extension tubes by vinyl adhesive. They contain a Luer lock for connecting with the gas generator line and the vacuum pump.

The temperature control system, very simply, as shown in Fig. 6 includes a controller 36, containing thermocouple thermometer 42 (such as Omega Model 660) which provides input to an Intel 8752 microcontroller (or other similar or equivalent microcontroller or electronic system) preferably having a keyboard or key input 43 and readouts 44a showing temperature, overtemperature situations, and the environment. The gas generator 44 (preferably Henes Products corporation Model MG-75) is attached via nylon tubing to the gas input hub; one needle valve 35, a solenoid valve 39, and a dump valve are attached within the line to the microcontroller 31 for control of the gas input. A booster solenoid 37 is placed in parallel to the main line for better temperature control. Finally software is provided to control the system according to the flow charts shown on Fig. 5.

Overall, the system provides a remarkably effective hot tip catheter. The use of a guidewire is eliminated, and dissociation of the tip from the catheter, a persistent problem with prior hot tip catheters, is avoided. Very high temperatures can be reached without catheter deterioration, and the preferred catheter has been found to reach 800°C· without deterioration or negative effect. Further, the catheter quite effectively maintains a constant temperature during operation. Thus the catheter has great potential for angioplasty and uses beyond it, for example where tissue vaporization is necessary, for discectomies, and the like.

It is to be understood, of course, that the above description is to be taken by way of illustration and not by way of limitation, and that various catheters, temperature control loops and other devices, methods, and apparatus will fall within the spirit and scope of the invention as defined by the appended claims.

All references, patents, and patent applications referred to herein are hereby incorporated by reference as though fully set forth.

## Claims

1. A catheter comprising a tip portion, and an elongated body portion having a distal and medial section for insertion in vivo, and a proximal section for extension external of the body being treated, said body portion having at least one lumen wherein said tip portion is distally bonded to the body portion to minimize tip dissociation,

2. A catheter comprising an elongated body portion having at least one central lumen, wherein said body portion is made of metal.

3. A catheter according to claim 2 and wherein said catheter includes a tip portion distally bonded to said body portion.

4. A catheter according to claim 1, 2 or 3 and wherein the tip and the body are made of material having a melting point above about 200° C.

5. A catheter according to claim 4 and wherein the tip and body are made from material selected from metals, ceramics, polyamides, carbon.

6. A catheter according to claim 5 and wherein the tip and the body are made of metal and welded together.

7. A catheter according to claim 6 and wherein the metal is stainless steel, and the catheter further includes a means for creating heat at the tip portion.

8. A catheter according to claim 7 and wherein the heat-creating means includes a gas inlet, vacuum outlet, and a catalyst adjacent the tip adapted for self-ignition in the presence of gas.

9. A catheter according to claim 8 further including means for controlling the temperature of the tip.

10. A catheter according to claim 9 and wherein said temperature controlling means includes tip-temperature measuring means and means for changing gas input in response to the tip temperature.

11. A catheter according to claim 10 and wherein said means for changing gas input includes:

a) a main gas input pathway and a parallel gas input pathway from a gas source to the catheter;

b) means for variably controlling gas input to the main pathway;

c) means for periodically opening and closing said parallel gas pathway;

d) means for closing all gas pathways to the catheter; and

e) means for purging the pathways of gas.

12. A catheter according to any preceding claim and wherein the body portion is spirally-wound.

13. A catheter according to claim 12 and wherein the body portion is formed of relatively flat wire, each spiral substantially adjacent the next, whereby column strength of the catheter is increased.

14. A catheter according to claim 13 and wherein the body portion includes sealing means along its length and at the distal tip to minimize leakage through the catheter body.

15. Apparatus comprising:
a metal tip portion;
an elongated body portion with at least one central lumen, said body portion consisting essentially of spirally wound metal wire sufficiently flat and sufficiently tightly wound to maximize column strength;
means for heating the tip portion, and wherein said body portion is welded to said tip portion whereby dissociation of the tip is minimized.

16. Apparatus according to claim 15 and wherein the tip heating means comprises a gas inlet lumen, a catalyst adjacent the tip for heating the tip portion by catalyzing a heat-producing reaction of the gas, a vacuum outlet lumen for removing by-products of the reaction, and a temperature control loop.

17. Apparatus according to claim 16 and including sealing means for said body portion.

18. Apparatus according to claim 17 and wherein said sealing means includes a polytetrafluoroethylene jacket for the body portion and a collar adjacent the tip and surrounding said jacket.

19. Apparatus according to claim 16, 17 or 18 and wherein said temperature control loop includes:

a) a tip-temperature reading means;

b) a gas generator and a main and a parallel pathway from the generator to the gas inlet lumen;

c) means for variably controlling the gas in one of said pathways;

d) means for periodically opening and closing the other of said pathways;

e) means for stopping the flow of gas to said catheter;

f) means for purging the pathways of gas; and

g) means for controlling means c), d), e) and f) in response to tip temperature.

20. A flow-control loop responsive to an external condition comprising:

a) means for reading the external condition;

b) a pathway between the flow source and the flow output;
and

c) means for variably controlling the flow through the pathway in response to readout of the external condition from the reading means.

21. A flow-control loop according to claim 20 and wherein the external condition is a condition of a catheter.

22. A flow-control loop according to claim 21 and wherein the flow is of gas and the external condition is the temperature of the tip of a hot tip catheter.

23. A flow-control loop according to any one of claims 20 to 22 and wherein said means for variably controlling the flow includes a needle valve, a stepper motor, and a controller for activating the motor to position the needle valve at a selected position in the pathway to control the flow in response to the external condition.

24. A flow-control loop according to claim any one of claims 20 to 23 and wherein said loop includes:

d) a second pathway parallel to said pathway;

e) a valve for periodically opening and closing said second pathway to increase the flow in response to said external variable; and

f) a controller for opening and closing said valve in response to said readout.

25. A flow control loop according to claim 24 wherein said valve is a one-way solenoid valve pulsed to intermittently permit more gas in the system, and further including:

g) a three-way solenoid valve for shutting said pathway;
and

h) a three-way solenoid valve for purging said pathway of gas.

Fig. 1

Fig. 3

FIG. 4

6a' 6a

5a

4

18

1

5b

7

1

1

3

1

1

5

6b' 6b

8

20a 20b

10

11

2

21

FIG. 2

EP 0 352 955 A2

| H/M RESET | COLD START ↓ CLOSE SHUTTER VALVE ↓ CLOSE BOOSTER ↓ ACCEPT TEMP. ↓ ACCEPT ENVIR. ↓ HOME MOTOR ↓ | FOOT SWITCH JUST ON | INITIALIZE ↓ CLOSE DUMP VALVE ↓ POSITION MOTOR ↓ CHECK OVER TEMP. ↓ CHECK PRESS. LIMIT ↓ CHECK VACUUM LIMIT ↓ |
|---|---|---|---|
| FOOT SWITCH ON | OPERATION ↓ OPEN SHUTTER VALVE ↓ CHECK OVER TEMP. ↓ CONTROL BOOSTER VALVE ↓ CHECK PRESS. LIMIT ↓ CHECK VACUUM LIMIT ↓ | SYSTEM ERROR FOOT SWITCH OFF | IDLE ↓ CLOSE SHUTTER VALVE ↓ OPEN DUMP VALVE ↓ STOP MOTOR ↓ CHECK OVER TEMP. ↓ CHECK PRESS. LIMIT ↓ CHECK VACUUM LIMIT |

FIG. 5

EP 0 352 955 A2

FIG. 6

EP 0 352 955 A2